# EUROPÄISCHE PATENTSCHRIFT

(11) **EP 0 345 294 B1**
(45) Veröffentlichungstag und Bekanntmachung des Hinweises auf die Patenterteilung: **27.04.1994**
(21) Anmeldenummer: 88902434.5
(22) Anmeldetag: 15.02.1988
(51) Int. Cl.: C07C 15/00, A61K 31/28

(54) **PLATINKOMPLEXE ENTHALTENDE ARZNEIMITTEL**
DRUGS CONTAINING PLATINUM COMPLEXES
MEDICAMENTS CONTENANT DES COMPLEXES DE PLATINE

(30) Priorität: 19.02.1987 CH 638/87
(43) Veröffentlichungstag der Anmeldung: 13.12.1989
(73) Patentinhaber: ASTA Medica Aktiengesellschaft, D-01277 Dresden (DE)
(72) Erfinder: KEPPLER, Bernhard, K., D-6830 Schwetzingen (DE); BLUM, Helmut, D-4000 Düsseldorf 13 (DE)
(86) Internationale Anmeldenummer: EP8800111
(87) Internationale Veröffentlichungsnummer: WO8806149

(56) Entgegenhaltungen:
- EP-A- 0 113 508
- EP-A- 0 155 705
- INORG. CHEM., 1986, 25, S. 726-734, American Chemical Society; T. G. APPLETON et al: NMR Spectra of iminobis(methylenephosphonic acid), HN(CH2PO3H2)2, and related ligands and of their complexes with platinum(II)"

## Beschreibung

Die Erfindung betrifft Platinkomplexe enthaltende Arzneimittel. Ein wesentliches Strukturelement der Platinkomplexe ist die Anwesenheit eines Liganden, der N- und Phosphonsäuregruppen enthält.

Phosphonsäuren werden nicht nur in der Technik, sondern auch für medizinische Zwecke eingesetzt, vgl. The Role of Phosphonates in Living Systems, CAC Press, Inc., Boca Raton, Florida (1982); M.D. Francis et al., J. Chem. Educ 55 (12), 760-766 (1978).

Es ist bekannt, daß sich bestimmte Phosphonsäuren nach intravenöser Verabreichung im Knochen- und Tumorgewebe anreichern. So wird beispielsweise die Pagetsche Knochenkrankheit mit dem Dinatriumsalz der Ethan-1-hydroxy-1,1-diphosphonsäure behandelt, vgl. The Merck Index, Merck & Co., Inc., Rahway, N.J., USA (1983) 3812. Die unter der INN-Bezeichnung Cisplatin bekannte Verbindung cis-Diammindichloroplatin(II) wird insbesondere zur Behandlung von Hodentumoren, Eierstocktumoren oder kleinzelligen Bronchialcarcinomen eingesetzt.

Aus der EP-A 0 155 705 sind zu therapeutischen Zwecken einsetzbare Platinverbindungen bekannt, die einerseits mit Diaminen und andererseits mit eine Phosphon- und eine Carboxylfunktion aufweisenden Liganden, insbesondere vom Typ der Phosphonoessigsäure, komplexiert sind.

Weiterhin sind in Inorg. Chem. 25, 726-734 (1986) Platinkomplexe beschrieben, die teilweise mit den Wirkstoffen der Arzneimittel der Erfindung übereinstimmen, ohne daß jedoch ihre Eignung als Arzneimittelwirkstoffe offenbart wird.

Es wurde nun gefunden, daß Arzneimittel mit einem Gehalt an Platinkomplexen der allgemeinen Formel I

[(R¹NH₂)ₙ Pt]_{q} Y Xₚ (I)

in welcher der Aminligand R¹NH₂ NH₃, Methylamin, Ethylamin, Propylamin, Isopropylamin, Butylamin, Hexylamin, Cyclohexylamin, Ethylendiamin, 1,2-Diaminopropan, cis-1,2-Diaminocyclohexan oder 1,1-Di(aminomethyl)cyclohexan ist,
Y
a) eine Phosphonsäure der allgemeinen Formel II

   R²-C(R³)(PO₃H₂)₂ (II)

   in der
   R² Wasserstoff, C₁-C₃-Alkyl, gegebenenfalls Hydroxyl-substituiertes C₅-C₆-Cycloalkyl, Phenyl, Hydroxyphenyl, Halogen, R⁴R⁵ N-(CH₂)ᵣ-, wobei R⁴ und R⁵, die gleich oder verschieden sein können, Wasserstoff und/oder C₁-C₃-Alkyl und r eine Zahl von 2 bis 4 bedeuten, oder -CH(COOH)-CH₂-COOH bedeutet, und
   R³ Amino Mono- oder Di-C₁-C₃-Alkylamino Hydroxy oder Chlor ist,
b) eine Phosphonsäure der allgemeinen Formel III

   R⁶-N(CH₂-PO₃H₂)₂ (III)

   in der R⁶ -CH₂-PO₃H₂, -CH₂-COOH oder
   -(CH₂)ₛ-N(CH₂-PO₃H₂)₂, wobei s eine Zahl von 2 bis 6 ist, bedeutet,
c) Iminobis(methylenphosphonsäure) oder
d) Ethylendiamintetramethylenphosphonsäure
e) Azacycloheptandiphosphonsäure,
X ein einfach negativ geladenes Anion,
n die Zahlen 1 oder 2, die Zahl 1 jedoch nur, falls R¹ eine an das Zentralmetall Pt koordinationsfähige Stickstoffunktion aufweist,
m die Zahl 1 oder 2, die Zahl 1 jedoch nur, wenn Y eine Verbindung der Formel II ist,
p die Zahlen 0 oder 2, die Zahl 2 jedoch nur, falls Pt in der Oxidationsstufe IV vorliegt,
und q die Zahl 1 oder 2, die Zahl 2 jedoch nur, wenn m die Zahl 1 ist,
oder
n die Zahl 0, und q die Zahl 1,
bedeuten,
oder deren Salze mit pharmakologisch annehmbaren Säuren und Basen.
vorteilhafte therapeutische Wirkungen aufweisen, die sie zur Behandlung von Krebskrankheiten, insbesondere von Knochentumoren, Osteosarkomen und Knochenmetastasen, Lebertumoren und Tumoren des retikulohistiozytären Systems (RHS) geeignet machen.

Die Platinkomplexe weisen infolge ihres Gehaltes an mindestens zwei Phosphonogruppen und einem N-Atom in dem Liganden Y eine größere Wasserlöslichkeit sowie eine höhere Bindungsfähigkeit auf Knochengewebe (Calciumhydroxylapatit) auf; dadurch erhalten die Platinkomplexe eine verbesserte Wirksamkeit im Vergleich zu mit Monophosphonsäuren komplexierten Verbindungen gemäß der EP-A 0 155 705.

Alkyl-, Alkenyl- und Alkoxyreste gemäß den obengenannten Definitionen können geradkettig oder verzweigt sein, wobei geradkettige Reste bevorzugt sind. Bevorzugte Halogenatome sind Chloratome.

Abhängig von dem pH-Wert des Reaktionsgemisches liegen die Phosphonsäuren im Reaktionsprodukt als Neutralliganden oder als einfach oder zweifach negativ geladene Liganden vor. Im ersten Fall erfolgt der Ladungsausgleich des Komplexes durch das Anion einer Säure, z.B. Chlorid. Im Falle des Vorliegens als negativ geladene Liganden erfolgt der Ladungsausgleich durch das Kation einer Base, vorzugsweise eines Alkalimetalls, insbesondere des Natriums.

Weiterhin bevorzugt sind Platinkomplexe bei denen die Liganden in cis-Stellung angeordnet sind.

Weiterhin bevorzugt sind Platinkomplexe, die zwei Phosphonsäuregruppen mit vicinaler und geminaler Stellung der Phosphonsäuregruppen enthalten. Besonders bevorzugt sind Verbindungen, die zwei geminale Phosphonsäuregruppen enthalten.

Die in den Platinkomplexen enthaltenen Phosphonsäuren sind bekannt und nach an sich bekannten Methoden erhältlich. Entsprechendes gilt für die Amine R¹NH₂.

Als Phosphonsäuren Y kommen beispielsweise die folgenden einschließlich der Salze derselben in Betracht (in Klammern sind die für diese Verbindungen üblichen Kurzbezeichnungen angegeben):
3-Amino-1-hydroxypropan-1,1-diphosphonsäure [APD],
Aminotris(methylenphosphonsäure) [ATMP],
Bis(dihydroxyphosphonylmethyl)aminoessigsäure [BPMAA],
Iminobis(methylenphosphonsäure) [IBM],
alpha-Amino(4-hydroxybenzyliden)diphosphonsäure [BDP3],
Methylaminomethandiphosphonsäure [MAD],
Aminoethandiphosphonsäure [AEDP],
Ethylendiamintetramethylenphosphonsäure [EDMP],
Azacycloheptandiphosphonsäure [AHP].

Anionen dieser Phosphonsäuren werden im folgenden durch ein an die Abkürzung angehängtes H mit tiefgestelltem negativem Index, der die Zahl der abgegebenen Protonen angibt, wiedergegeben. So bedeutet z.B. APDH₋₂ das zweifach negativ geladene Anion von APD.

Als Aminligand R¹NH₂ kommen die folgenden Amine in Betracht:
Ammoniak,
Methylamin,
Ethylamin,
Propylamin,
Isopropylamin,
Butylamin,
Hexylamin,
Ethylendiamin,
1,2-Diaminopropan,
cis-1,2-Diaminocyclohexan,
Cyclohexylamin,
1,1-Bis(aminomethyl)cyclohexan.

Bevorzugte Amine R¹NH₂ sind Ammoniak, Ethylamin, Propylamin, Isopropylamin, Ethylendiamin, 1,2-Diaminopropan, cis-1,2-Diaminocyclohexan und 1,1-Bis(aminomethyl)cyclohexan.

Als einfach negativ geladenes Anion X kommen beispielsweise neben Hydroxy und Chlorid auch Bromid, Jodid, Fluorid, Nitrat, Hydrogensulfat, Dihydrogenphosphat oder Anionen von organischen Säuren, wie beispielsweise Essigsäure, Propionsäure, Adipinsäure, Zitronensäure, Gluconsäure, Benzoesäure, Buttersäure, Maleinsäure, Laurinsäure, Malonsäure, Furmarsäure, Oxalsäure, Weinsäure, Stearinsäure, 2-Hydroxy-3-naphthalinsäure, Cyclobutandicarbonsäure oder Benzoldi- und tricarbonsäuren, wie beispielsweise 1,2,4-Benzoltricarbonsäure in Betracht.

Die Platinkomplexe können erhalten werden, indem man eine Verbindung der allgemeinen Formel

PtX₄

in der X wie oben definiert ist, mit Phosphonsäuren der allgemeinen Formel (II) oder (III) bzw. mit Iminobis(methylenphosphonsäure), Ethylendiamintetramethylenphosphonsäure oder Azacycloheptandiphosphonsäure umsetzt und gegebenenfalls reduziert, sowie das Reaktionsprodukt gewünschtenfalls in die Salze von pharmakologisch annehmbaren Säuren und Basen überführt.

Die Umsetzung wird vorzugsweise in wäßriger Lösung in einem Temperaturbereich von 5° bis 90°C, vorzugsweise bei 15 bis 60°C durchgeführt.

Die erfindungsgemäßen Arzneimittel werden vor allem intravenös, aber auch intramuskulär, intraperitoneal, subkutan oder peroral verabreicht. Auch eine äußerliche Applikation ist möglich. Bevorzugt ist die Verabreichung durch intravenöse Injektion oder intravenöse Infusion.

Die Arzneimittel werden nach an sich bekannten Verfahren hergestellt, wobei die Platinkomplexe als solche oder gegebenenfalls in Kombination mit geeigneten pharmazeutischen Trägerstoffen eingesetzt werden. Enthalten die neuen pharmazeutischen Zubereitungen neben dem Wirkstoff pharmazeutische Trägerstoffe, beträgt der Wirkstoffgehalt dieser Mischungen 0,1 bis 99,5, vorzugsweise 0,5 bis 95 Gew.-% der Gesamtmischung.

In Übereinstimmung mit der Erfindung wird ein Wirkstoff in jeder geeigneten Formulierung angewandt unter der Voraussetzung, daß die Ausbildung bzw. Aufrechterhaltung von ausreichenden Wirkstoffspiegeln gewährleistet ist. Das kann beispielsweise durch orale oder parenterale Gabe in geeigneten Dosen erreicht werden. Vorteilhafterweise liegt die pharmazeutische Zubereitung des Wirkstoffs in Form von Einheitsdosen vor, die auf die gewünschte Verabreichung abgestimmt sind. Eine Einheitsdosis kann zum Beispiel eine Tablette, ein Dragee, eine Kapsel, ein Suppositorium oder eine gemessene Volumenmenge eines Pulvers, eines Granulates, einer Lösung, einer Emulsion oder einer Suspension sein.

Unter "Einheitsdosis" im Sinne der vorliegenden Erfindung wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z.B. in Form einer Tablette mit Bruchkerbe.

Die pharmazeutischen Zubereitungen gemäß der Erfindung können, wenn sie in Einheitsdosen vorliegen und für die Applikation z.B. am Menschen bestimmt sind, etwa 0,1 bis 500 mg, vorteilhafterweise 10 bis 200 mg und insbesondere 50 bis 150 mg Wirkstoff enthalten.

Im allgemeinen werden in der Humanmedizin der oder die Wirkstoffe bei oraler Gabe in einer Tagesdosis von 0,1 bis 5, vorzugsweise 1 bis 3 mg/kg Körpergewicht, gegebenenfalls in Form mehrerer, vorzugsweise 1 bis 3 Einzelgaben zur Erzielung der gewünschten Ergebnisse verabreicht. Eine Einzelgabe enthält den oder die Wirkstoffe in Mengen von 0,1 bis 5, vorzugsweise 1 bis 3 mg/kg Körpergewicht. Bei einer oralen Behandlung können ähnliche Dosierungen zur Anwendung kommen.

Die therapeutische Verabreichung der pharmazeutischen Zubereitung kann 1 bis 4 mal am Tage zu festgelegten oder variierenden Zeitpunkten erfolgen, z.B. jeweils vor den Mahlzeiten und/oder am Abend. Es kann jedoch erforderlich sein, von den genannten Dosierungen abzuweichen, und zwar in Abhängigkeit von der Art, dem Körpergewicht und dem Alter des zu behandelnden Individuums, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Es kann sich auch als zweckmäßig erweisen, das Arzneimittel nur einmalig oder im Abstand von mehreren Tagen zu verabreichen.

Die Festlegung der jeweils erforderlichen optimalen Dosierung und Applikationsart der Wirkstoffe kann durch jeden Fachmann aufgrund seines Fachwissens erfolgen.

Die pharmazeutischen Zubereitungen bestehen in der Regel aus den erfindungsgemäßen Wirkstoffen und nichttoxischen, pharmazeutisch verträglichen Arzneimittelträgern, die als Zumischung oder Verdünnungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z.B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Zur oralen Anwendung können z.B. Tabletten, Dragees, harte und weiche Kapseln, z.B. aus Gelatine, dispergierbare Pulver, Granulate, wäßrige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Lactose; Granulierungs- und Verteilungsmittel, z.B. Maisstärke oder Alginate; Bindemittel, z.B. Stärke, Gelatine oder Akaziengummi und Gleitmittel, z.B. Aluminium- oder Magnesiumstearat, Talkum oder Silikonöl, enthalten. Sie können zusätzlich mit einem Überzug versehen sein, der auch so beschaffen sein kann, daß er eine verzögerte Auflösung und Resorption des Arzneimittels im Gastrointestinaltrakt bewirkt, so daß z.B. eine bessere Verträglichkeit, Protahierung oder eine Retardierung erreicht wird.

Gelatinekapseln können den Arzneistoff vermischt mit einem festen, z.B. Calciumcarbonat oder Kaolin, oder einem öligen, z.B. Oliven-, Erdnuß- oder Paraffinöl, Verdünnungsmittel enthalten.

Wäßrige Suspensionen, die gegebenenfalls kurzfristig zubereitet werden, können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycetanol, Polyoxyethylensorbitolmonooleat, Polyoxyethylensorbitanmonooleat oder Lecithin; Konservierungsmittel, z.B. Methyl- oder Propylhydroxybenzoate; Geschmacksmittel; Süßungsmittel, z.B. Saccharose, Lactose, Natriumcyclamat, Dextrose, Invertzuckersirup, enthalten.

Ölige Suspensionen können z.B. Erdnuß-, Oliven-, Sesam-, Kokos- oder Paraffinöl und Verdickungsmittel, wie z.B. Bienenwachs, Hartparaffin oder Cetylalkohol, enthalten; ferner Süßungsmittel, Geschmacksmittel und Antioxidantien.

In Wasser dispergierbare Pulver und Granulate können eine erfindungsgemäße Verbindung in Mischung mit Dispergier-, Benetzungs- und Suspendiermitteln, z.B. den obengenannten, sowie mit Süßungsmitteln, Geschmacksmitteln und Farbstoffen enthalten.

Emulsionen können z.B. Oliven-, Erdnuß- oder Paraffinöl neben Emulgiermitteln, wie z.B. Akaziengummi, Traganthgummi, Phosphatiden, Sorbitanmonooleat, Polyoxyethylensorbitanmonooleat, und Süßungs- und Geschmacksmitteln enthalten.

Die Wirkstoffe können gegebenenfalls mit einem oder mehreren der angegebenen Träger- oder Zusatzstoffe auch in mikroverkapselter Form formuliert werden.

Die Herstellung der Wirkstoffe der Arzneimittel der Erfindung wird im folgenden anhand bevorzugter Ausführungsbeispiele näher erläutert.

Für die Herstellung der folgenden Beispiele verwendet man zweckmäßigerweise eine aktivierte Cisplatin-Lösung, die wie folgt hergestellt wird:
1 mmol Cisplatin werden in Wasser gelöst; dann gibt man 0,98 mmol Ag₂SO₄, in Wasser gelöst, hinzu. Anschließend schüttelt man 24 h lichtgeschützt bei Raumtemperatur und filtriert AgCl ab. 0,96 mmol Ba(OH)₂ werden in Wasser gelöst und zugegeben. Danach wird 24 h lichtgeschützt bei Raumtemperatur geschüttelt und BaSO₄ wird abfiltriert. Die resultierende Lösung ist aktivierte Cisplatin-Lösung.

### Beispiel 1.

### cis[Pt(NH₃)₂(Na₂-APDH₋₁)(OH) x 2H₂O

2 mmol Na₂APD x 2H₂O werden in 50 ml H₂O gelöst; dann werden 2 mmol aktivierte Cisplatin-Lösung zugegeben, es entsteht eine leichte Trübung. Anschließend wird bei 50°C abrotiert. Hierbei entsteht eine klare gelbe Lösung. Nach vollständigem Abrotieren des Lösungsmittels verbleibt ein elfenbeinfarbenes Pulver, das über einem Trockenmittel getrocknet wird.

### Beispiel 2.

### cis[Pt(NH₃)₂(MADH₋₂)]

4 mmol MAD werden in 50 ml H₂O gelöst. Hierzu werden 4 mmol aktivierte Cisplatin-Lösung gegeben, dabei entsteht eine weiße, flockige Trübung, die nicht filtrierbar ist. Dann wird auf 50°C erhitzt, es entsteht eine klare gelbe Lösung.

Das Lösungsmittel wird abrotiert, und man erhält ein grün gefärbtes Produkt, das über einem Trockenmittel getrocknet wird.

### Beispiel 3.

### cis[Pt(NH₃)₂(BDP3H₋₂)] x 3H₂O

Es werden 4 mmol BDP-3 in 100 ml H₂O gelöst. Anschließend gibt man 4 mmol aktivierte Cisplatin-Lösung hinzu und erhält einen weißen Niederschlag, welcher abgesaugt, mit Wasser gewaschen und anschließend über einem Trockenmittel getrocknet wird.

### Beispiel 4.

### cis[Pt(NH₃)₂(ATMPH₋₂)]

Es werden 2 mmol ATMP in 25 ml H₂O gelöst und anschließend 2 mmol aktivierte Cisplatin-Lösung zugegeben. Man läßt die Lösung eine Stunde bei 50°C rühren, rotiert ab und erhält die Verbindung als cremefarbenes Pulver.

### Beispiel 5.

### cis[Pt(NH₃)₂(BPMAAH₋₂)]

2 mmol BPMAA werden in 30ml H₂O gelöst. Anschließend werden 2 mmol aktivierte Cisplatin-Lösung zugegeben und eine Stunde bei 50°C gerührt. Anschließend wird abrotiert und der Rückstand über einem Trockenmittel getrocknet.

### Beispiel 6.

### cis[Pt(Cl₂)(ATMP/ATMPH₋₁)₂]

2 mmol PtCl₄ werden in 20 ml H₂O gelöst, dazu gibt man 4 mmol ATMP, gelöst in 40 ml H₂O und läßt 3 Stunden bei 50°C lichtgeschützt rühren. Anschließend wird die Lösung abrotiert und zwei Stunden am HV bei 50°C getrocknet. Man erhält eine dunkelgrüne hygroskopische Substanz.

### Beispiel 7.

### cis[PtCl₂(BPMAA/BPMAAH₋₁)₂]

1 mmol PtCl₄ werden in 10 ml H₂O gelöst. Dazu gibt man 2 mmol BPMAA, gelöst in 20 ml H₂O. Die Lösung wird 1 Stunde bei 50°C am Rückfluß erhitzt, anschließend abrotiert und zwei Stunden bei 50°C am HV getrocknet. Man erhält eine orangebraune Substanz.

### Beispiel 8.

### cis[PtCl₂(BDP3)₂]

4 mmol BDP-3 werden in 200 ml H₂O gelöst, dazu gibt man 2 mmol PtCl₄, gelöst in 20 ml H₂O und läßt 3 Stunden bei 50°C rühren. Anschließend wird abrotiert und zwei Stunden bei 50°C am HV getrocknet und zusätzlich im Exsiccator über einem Trockenmittel und KOH über Nacht getrocknet. Man erhält eine grüne Substanz.
Der analoge (BDP4)-Pt-Komplex wird entsprechend hergestellt.

### Beispiel 9.

### cis-[Diammino-azocycloheptan-2,2-diphosphonato-platin(II)] cis[Pt(AHP)(NH₃)₂]

2 Mol AHP wurden in 150 ml Wasser unter Erwärmen gelöst. 25 ml einer aktivierten Cisplatinlösung (2 mmol) wurden zugegeben. Die Lösung wurde gelb und allmählich trübe. Sie wurde abrotiert und über einem Trockenmittel getrocknet. Die grau-grüne Verbindung wurde durch Elementaranalyse und 31P-NMR-Spektroskopie charakterisiert.

### Beispiel 10.

### cis-[Imino-bis(methylenphosphonato)diammino-platin(II)] cis[Pt(IBM)(NH₃)₂]

2 mmol IBM wurden in 50 ml Wasser gelöst. 25 ml einer aktivierten Cisplatinlösung (2 mmol) wurden zugegeben; die Lösung wurde hellgelb und leicht trübe. Nach 1 h Rühren bei 50°C wurde abrotiert und das Produkt über einem Trockenmittel getrocknet. Die cremeweiße Verbindung wurde durch Elementaranalyse und durch ³¹PMR-Spektroskopie charakterisiert.

### Beispiel 11

### Dinatriumdiammin(dichlormethandiphosphonato)platinat(II), Na₂[(NH₃)₂Pt(Cl₂-MDPH₋₄)]

Die Titelverbindung wird durch Umsetzung von Na₂MDPH₋₂ mit aktivierter Cisplatin-Lösung hergestellt. Man erhält ein sandfarbenes Produkt.

## Patentansprüche

1. Arzneimittel, enthaltend Platinkomplexe der allgemeinen Formel I
[(R¹NH₂)ₙ Pt]_{q} YₘXₚ (I)
in welcher der Aminligand R¹NH₂
NH₃, Methylamin, Ethylamin, Propylamin, Isopropylamin, Butylamin, Hexylamin, Cyclohexylamin, Ethylendiamin, 1,2-Diaminopropan, cis-1,2-Diaminocyclohexan oder 1,1-Bis(aminomethyl)cyclohexan ist,
Y
a) eine Phosphonsäure der allgemeinen Formel II
R²-C(R³)(PO₃H₂)₂ (II)
in der
R² Wasserstoff, C₁-C₃-Alkyl, gegebenenfalls Hydroxyl-substituiertes C₅-C₆-Cycloalkyl, Phenyl, Hydroxyphenyl, Halogen, R⁴R⁵N-(CH₂)ᵣ-, wobei R⁴ und R⁵, die gleich oder verschieden sein können, Wasserstoff und/oder C₁-C₃-Alkyl und r eine Zahl von 2 bis 4 bedeuten, oder -CH(COOH)-CH₂-COOH bedeutet, und
R³ Amino, Mono- oder Di-C₁-C₃-Alkylamino Hydroxy oder Chlor ist,
b) eine Phosphonsäure der allgemeinen Formel III
R⁶-N(CH₂-PO₃H₂)₂ (III)
in der R⁶ -CH₂-PO₃H₂, -CH₂-COOH oder
-(CH₂)ₛ-N(CH₂-PO₃H₂)₂, wobei s eine Zahl von 2 bis 6 ist, bedeutet,
c) Iminobis(methylenphosphonsäure) oder
d) Ethylendiamintetramethylenphosphonsäure
e) Azacycloheptandiphosphonsäure,
X ein einfach negativ geladenes Anion,
n die Zahlen 1 oder 2, die Zahl 1 jedoch nur, falls R¹ eine an das Zentralmetall Pt koordinationsfähige Stickstoffunktion aufweist,
m die Zahl 1 oder 2, die Zahl 1 jedoch nur, wenn Y eine Verbindung der Formell II ist,
p die Zahlen 0 oder 2, die Zahl 2 jedoch nur, falls Pt in der Oxidationsstufe IV vorliegt,
und q die Zahl 1 oder 2, die Zahl 2 jedoch nur, wenn m die Zahl 1 ist,
oder
n die Zahl 0, und q die Zahl 1,
bedeuten,
oder deren Salze mit pharmakologisch annehmbaren Säuren und Basen.

2. Arzneimittel nach Anspruch 1, dadurch gekennzeichnet, daß die Platinkomplexe als Natriumsalze vorliegen.

3. Arzneimittel nach Anspruch 1 oder 2, wobei Y aus der Gruppe, bestehend aus 3-Amino-1-hydroxypropan-1,1-diphosphonsäure [APD], Aminotris(methylenphosphonsäure) [ATMP], Bis(dihydroxyphosphonylmethyl)aminoessigsäure [BPMAA], Iminobis(methylenphosphonsäure) [IBM], alpha-Amino(4-hydroxybenzyliden)diphosphonsäure [BDP3], Methylaminomethandiphosphonsäure [MAD], Aminoethandiphosphonsäure [AEDP], Ethylendiamintetramethylenphosphonsäure [EDMP], Azacycloheptandiphosphonsäure [AHP], ausgewählt ist.

4. Arzneimittel nach mindestens einem der Ansprüche 1 bis 3, wobei der Platinkomplex
cis[Pt(NH₃)₂(Na₂-APDH₋₁)(OH) x 2H₂O
ist.

5. Arzneimittel nach mindestens einem der Ansprüche 1 bis 3, wobei der Platinkomplex
cis[Pt(NH₃)₂(MADH₋₂)]
ist.

6. Arzneimittel nach mindestens einem der Ansprüche 1 bis 3, wobei der Platinkomplex
cis[Pt(NH₃)₂(BDP3H₋₂)] x 3H₂O
ist.

7. Arzneimittel nach mindestens einem der Ansprüche 1 bis 3, wobei der Platinkomplex
cis[Pt(NH₃)₂(ATMPH₋₂)]
ist.

8. Arzneimittel nach mindestens einem der Ansprüche 1 bis 3, wobei der Platinkomplex
cis[Pt(NH₃)₂(BPMAAH₋₂)]
ist.

9. Arzneimittel nach mindestens einem der Ansprüche 1 bis 3, wobei der Platinkomplex
cis[Pt(Cl₂)(ATMP/ATMPH₋₁)₂]
ist.

10. Arzneimittel nach mindestens einem der Ansprüche 1 bis 3, wobei der Platinkomplex
cis[PtCl₂(BPMAA/BPMAAH₋₁)₂]
ist.

11. Arzneimittel nach mindestens einem der Ansprüche 1 bis 3, wobei der Platinkomplex
cis[PtCl₂(BDP3)₂]
ist.

12. Arzneimittel nach mindestens einem der Ansprüche 1 bis 3, wobei der Platinkomplex
cis[Pt(AHP)(NH₃)₂]
ist.

13. Arzneimittel nach mindestens einem der Ansprüche 1 bis 3, wobei der Platinkomplex
cis[Pt(IBM)(NH₃)₂]
ist.

14. Arzneimittel nach mindestens einem der Ansprüche 1 bis 13, enthaltend weiterhin einen nichttoxischen, pharmazeutisch verträglichen Arzneimittelträger.

15. Verfahren zur Herstellung von Platinphosphonsäurekomplexen der allgemeinen Formel I
[(R¹NH₂)ₙ Pt]_{q} YₘXₚ (I)
in welcher der Aminligand R¹NH₂
NH₃, Methylamin, Ethylamin, Propylamin, Isopropylamin, Butylamin, Hexylamin, Cyclohexylamin, Ethylendiamin, 1,2-Diaminopropan, cis-1,2-Diaminocyclohexan oder 1,1-Bis(aminomethyl)cyclohexan ist,
Y
a) eine Phosphonsäure der allgemeinen Formel II
R²-C(R³)(PO₃H₂)₂ (II)
in der
R² Wasserstoff, C₁-C₃-Alkyl, gegebenenfalls Hydroxyl-substituiertes C₅-C₆-Cycloalkyl, Phenyl, Hydroxyphenyl, Halogen, R⁴R⁵N-(CH₂)ᵣ-, wobei, R⁴ und R⁵, die phenyl, Halogen, R⁴R⁵N-(CH₂)ᵣ-, wobei R⁴ und R⁵, die gleich oder verschieden sein können, Wasserstoff und/oder C₁-C₃-Alkyl und r eine Zahl von 2 bis 4 bedeuten, oder -CH(COOH)-CH₂-COOH bedeutet, und
R³ Amino oder Mono- oder Di-C₁-C₃-Alkylamino ist,
b) eine Phosphonsäure der allgemeinen Formel III
R⁶-N(CH₂-PO₃H₂)₂ (III)
in der R⁶ -CH₂-PO₃H₂, -CH₂-COOH oder
-(CH₂)ₛ-N(CH₂-PO₃H₂)₂, wobei s eine Zahl von 2 bis 6 ist, bedeutet,
c) Iminobis(methylenphosphonsäure) oder
d) Ethylendiamintetramethylenphosphonsäure
e) Azacycloheptandiphosphonsäure,
X ein einfach negativ geladenes Anion,
n die Zahlen 1 oder 2, die Zahl 1 jedoch nur, falls R¹ eine an das Zentralmetall Pt koordinationsfähige Stickstoffunktion aufweist,
m die Zahl 1,
p die Zahlen 0 oder 2, die Zahl 2 jedoch nur, falls Pt in der Oxidationsstufe IV vorliegt,
und q die Zahl 1 oder 2,
oder
n die Zahl 0, m die Zahl 2 und q die Zahl 1,
bedeuten,
oder deren Salzen mit pharmakologisch annehmbaren Säuren und Basen, dadurch gekennzeichnet, daß man eine Verbindung der allgemeinen Formel
PtX₄
in der X wie oben definiert ist, mit Phosphonsäuren der allgemeinen Formel (II) oder (III) bzw. mit Iminobis(methylenphosphonsäure), Ethylendiamintetramethylenphosphonsäure oder Azacycloheptandiphosphonsäure umsetzt und gegebenenfalls reduziert, sowie das Reaktionsprodukt gewünschtenfalls in die Salze von pharmakologisch annehmbaren Säuren und Basen überführt.

## Claims

1. Drugs containing platinum complexes of general formula I:
[(R¹NH₂)ₙPt]_{q}YₘXₚ (I)
in which the amine ligand R¹NH₂ is NH₃, methylamine, ethylamine, propylamine, isopropylamine, butylamine, hexylamine, cyclohexylamine, ethylenediamine, 1,2-diaminopropane, cis-1,2-diaminocyclohexane or 1,1-bis(aminomethyl)cyclohexane,
Y is
a) a phosphonic acid of general formula II:
R²-C(R³)(PO₃H₂)₂ (II)
in which
R² is hydrogen, C₁-C₃-alkyl, optionally hydroxy-substituted C₅-C₆-cycloalkyl, phenyl, hydroxyphenyl, halogen, R⁴R⁵N-(CH₂)ᵣ-, R⁴ and R⁵, which can be identical or different, being hydrogen and/or C₁-C₃-alkyl and r being a number from 2 to 4, or -CH(COOH)-CH₂-COOH, and
R³ is amino, mono- or di-C₁-C₃-alkylamino, hydroxy or chlorine,
b) a phosphonic acid of general formula III:
R⁶-N(CH₂-PO₃H₂)₂ (III)
in which R⁶ is -CH₂-PO₃H₂, -CH₂-COOH or -(CH₂)ₛ- N(CH₂-PO₃H₂)₂, s being a number from 2 to 6,
c) iminobis(methylenephosphonic acid),
d) ethylenediaminetetramethylenephosphonic acid or
e) azacycloheptanediphosphonic acid,
X is a singly negatively charged anion,
n is the number 1 or 2, being the number 1 only if R¹ has a nitrogen group capable of coordination to the central Pt metal,
m is the number 1 or 2, being the number 1 only if Y is a compound of formula II,
p is the number 0 or 2, being the number 2 only if Pt is present in oxidation state IV, and
q is the number 1 or 2, being the number 2 only if m is the number 1, or
n is the number 0 and q is the number 1,
or salts thereof with pharmacologically acceptable acids and bases.

2. Drugs according to Claim 1, characterized in that the platinum complexes are present as sodium salts.

3. Drugs according to Claim 1 or 2 in which Y is selected from the group consisting of 3-amino-1- hydroxypropane-1,1-diphosphonic acid [APD], aminotris(methylenephosphonic acid) [ATMP], bis(dihydroxyphosphonylmethyl)aminoacetic acid [BPMAA], iminobis(methylenephosphonic acid) [IBM], alpha-amino(4-hydroxybenzylidene)diphosphonic acid [BDP3], methylaminomethanediphosphonic acid [MAD], aminoethanediphosphonic acid [AEDP], ethylenediaminetetramethylenephosphonic acid [EDMP] and azacycloheptanediphosphonic acid [AHP].

4. Drugs according to at least one of Claims 1 to 3 in which the platinum complex is
cis-[Pt(NH₃)₂(Na₂-APDH₋₁)(OH)]·2H₂O

5. Drugs according to at least one of Claims 1 to 3 in which the platinum complex is
cis-[Pt(NH₃)₂(MADH₋₂)]

6. Drugs according to at least one of Claims 1 to 3 in which the platinum complex is
cis-[Pt(NH₃)₂(BDP3H₋₂)]·3H₂O

7. Drugs according to at least one of Claims 1 to 3 in which the platinum complex is
cis-[Pt(NH₃)₂(ATMPH₋₂)]

8. Drugs according to at least one of Claims 1 to 3 in which the platinum complex is
cis-[Pt(NH₃)₂(BPMAAH₋₂)]

9. Drugs according to at least one of Claims 1 to 3 in which the platinum complex is
cis-[Pt(Cl₂)(ATMP/ATMPH₋₁)₂]

10. Drugs according to at least one of Claims 1 to 3 in which the platinum complex is
cis-[PtCl₂(BPMAA/BPMAAH₋₁)₂]

11. Drugs according to at least one of Claims 1 to 3 in which the platinum complex is
cis-[PtCl₂(BDP3)₂]

12. Drugs according to at least one of Claims 1 to 3 in which the platinum complex is
cis-[Pt(AHP)(NH₃)₂]

13. Drugs according to at least one of Claims 1 to 3 in which the platinum complex is
cis-[Pt(IBM)(NH₃)₂]

14. Drugs according to at least one of Claims 1 to 13 which also contain a non-toxic, pharmaceutically acceptable excipient.

15. A process for the preparation of platinum/phosphonic acid complexes of general formula I:
[(R¹NH₂)ₙPt]_{q}YₘXₚ (I)
in which the amine ligand R¹NH₂ is NH₃, methylamine, ethylamine, propylamine, isopropylamine, butylamine, hexylamine, cyclohexylamine, ethylenediamine, 1,2-diaminopropane, cis-1,2-diaminocyclohexane or 1,1-bis(aminomethyl)cyclohexane,
Y is
a) a phosphonic acid of general formula II:
R²-C(R³)(PO₃H₂)₂ (II)
in which
R² is hydrogen, C₁-C₃-alkyl, optionally hydroxy-substituted C₅-C₆-cycloalkyl, phenyl, hydroxyphenyl, halogen, R⁴R⁵N-(CH₂)ᵣ-, R⁴ and R⁵, which can be identical or different, being hydrogen and/or C₁-C₃- alkyl and r being a number from 2 to 4, or -CH(COOH)-CH₂-COOH, and
R³ is amino or mono- or di-C₁- C₃-alkylamino,
b) a phosphonic acid of general formula III:
R⁶-N(CH₂-PO₃H₂)₂ (III)
in which R⁶ is -CH₂-PO₃H₂, -CH₂-COOH or -(CH₂)ₛ- N(CH₂-PO₃H₂)₂, s being a number from 2 to 6,
c) iminobis(methylenephosphonic acid),
d) ethylenediaminetetramethylenephosphonic acid or
e) azacycloheptanediphosphonic acid,
X is a singly negatively charged anion,
n is the number 1 or 2, being the number 1 only if R¹ has a nitrogen group capable of coordination to the central Pt metal,
m is the number 1,
p is the number 0 or 2, being the number 2 only if Pt is present in oxidation state IV, and
q is the number 1 or 2,
or
n is the number 0, m is the number 2 and q is the number 1,
or
salts thereof with pharmacologically acceptable acids and bases, characterized in that a compound of the general formula
PtX₄
in which X is as defined above, is reacted with phosphonic acids of general formula (II) or (III) or with iminobis(methylenephosphonic acid), ethylenediaminetetramethylenephosphonic acid or azacycloheptanediphosphonic acid and the reaction product is optionally reduced and, if desired, converted to the salts of pharmacologically acceptable acids and bases.

## Revendications

1. Médicaments, contenant des complexes de platine de formule générale I
[(R¹NH₂)ₙ Pt]_{q} YₘXₚ (I)
dans laquelle
le ligand aminé R¹NH₂ est un groupement NH₃, méthylamine, éthylamine, propylamine, isopropylamine, butylamine, hexylamine, cyclohexylamine, éthylènediamine, 1,2-diaminopropane, cis-1,2-diaminocyclohexane ou 1,1-bis(aminométhyl)cyclohexane,
Y représente
a) un groupement acide phosphonique de formule générale II
R²-C(R³)(PO₃H₂)₂ (II)
dans laquelle
R² est mis pour un atome d'hydrogène, pour un reste alkyle en C₁-C₃, cycloalkyle en C₅-C₆ éventuellement substitué par un groupement hydroxyle, phényle, hydroxyphényle, pour un atome d'halogène, pour R⁴R⁵N-(CH₂)ᵣ- (R⁴ et R⁵, qui peuvent être identiques ou différents, représentant des atomes d'hydrogène et/ou des restes alkyle en C₁-C₃ et r étant un nombre de 2 à 4) ou pour -CH(COOH)-CH₂-COOH, et
R³ est mis pour un groupement amino, mono- ou di(alkyl en C₁-C₃)amino, hydroxy ou pour un atome de chlore,
b) un groupement acide phosphonique de formule générale III
R⁶-N(CH₂-PO₃H₂)₂ (III)
dans laquelle
R⁶ est mis pour -CH₂-PO₃H₂, -CH₂-COOH ou -(CH₂)ₛ-N(CH₂-PO₃H₂)₂, s étant un nombre de 2 à 6,
c) un groupement imino-bis(acide méthylènephosphonique),
d) un groupement acide éthylènediaminetétraméthylènephosphonique ou
e) un groupement acide azacycloheptanediphosphonique,
X représente un anion à simple charge négative
n est mis pour le nombre 1 ou 2, mais n'est mis que pour le nombre 1 quand R¹ présente une fonction azotée susceptible de coordination sur le métal central Pt,
m est mis pour le nombre 1 ou 2, mais n'est mis que pour le nombre 1 quand Y est un composé de formule
p est mis pour le nombre 0 ou 2, mais n'est mis que pour le nombre 2 quand Pt est présent au degré d'oxydation IV, et
q est mis pour le nombre 1 ou 2, mais n'est mis que pour le nombre 2 quand m est le nombre 1,
ou
n est mis pour le nombre 0 et q pour le nombre 1,
ou leurs sels avec des acides et des bases acceptables pharmacologiquement.

2. Médicaments selon la revendication 1, caractérisés en ce que les complexes de platine sont présents sous forme de sels de sodium.

3. Médicaments selon la revendication 1 ou 2, dans lesquels Y est choisi dans le groupe constitué par l'acide 3-amino-1-hydroxypropane-1,1-diphosphonique [APD], l'amino-tris(acide méthylènephosphonique) [ATMP], l'acide bis(dihydroxyphosphonylméthyl)aminoacétique [BPMAA], l'imino-bis(acide méthylènephosphonique) [IBM], l'acide α-amino(4-hydroxybenzylidène)diphosphonique [BDP3], l'acide méthylaminométhanediphosphonique [MAD], l'acide aminoéthanediphosphonique [AEDP], l'acide éthylènediaminetétraméthylènephosphonique [EDMP] et l'acide azacycloheptanediphosphonique [AHP].

4. Médicaments selon l'une quelconque des revendications 1 à 3, dans lesquels le complexe de platine est
cis[Pt(NH₃)₂(Na₂-APDH₋₁)(OH) × 2H₂O

5. Médicaments selon l'une quelconque des revendications 1 à 3, dans lesquels le complexe de platine est
cis[Pt(NH₃)₂(MADH₋₂)]

6. Médicaments selon l'une quelconque des revendications 1 a 3, dans lesquels le complexe de platine est
cis[Pt(NH₃)₂-(BDP3H₋₂)] × 3H₂O

7. Médicaments selon l'une quelconque des revendications 1 à 3, dans lesquels le complexe de platine est
cis[Pt(NH₃)₂(ATMPH₋₂)]

8. Médicaments selon l'une quelconque des revendications 1 à 3, dans lesquels le complexe de platine est
cis[Pt(NH₃)₂(BPMAAH₋₂)]

9. Médicaments selon l'une quelconque des revendications 1 à 3, dans lesquels le complexe de platine est
cis[Pt(Cl₂)(ATMP/ATMPH₋₁)₂]

10. Médicaments selon l'une quelconque des revendications 1 à 3, dans lesquels le complexe de platine est
cis[PtCl₂(BPMAA/BPMAAH₋₁)₂]

11. Médicaments selon l'une quelconque des revendications 1 à 3, dans lesquels le complexe de platine est
cis[PtCl₂(BDP3)₂]

12. Médicaments selon l'une quelconque des revendications 1 à 3, dans lesquels le complexe de platine est
cis[Pt(AHP)(NH₃)₂]

13. Médicaments selon l'une quelconque des revendications 1 à 3, dans lesquels le complexe de platine est
cis[Pt(IBM)(NH₃)₂]

14. Médicaments selon l'une quelconque des revendications 1 à 13, contenant en outre un véhicule non toxique, acceptable pharmaceutiquement.

15. Procédé de préparation de complexes de platine-acide phosphonique de formule générale I
[(R¹NH₂)ₙ Pt]_{q} YₘXₚ (I)
dans laquelle
le ligand aminé R¹NH₂ est un groupement NH₃, méthylamine, éthylamine, propylamine, isopropylamine, butylamine, hexylamine, cyclohexylamine, éthylènediamine, 1,2-diaminopropane, cis-1,2-diaminocyclohexane ou 1,1-bis(aminomethyl)cyclohexane,
Y représente
a) un groupement acide phosphonique de formule générale II
R²-C(R³)(PO₃H₂)₂ (II)
dans laquelle
R² est mis pour un atome d'hydrogène, pour un reste alkyle en C₁-C₃, cycloalkyle en C₅-C₆ éventuellement substitué par un groupement hydroxyle, phényle, hydroxyphényle, pour un atome d'halogène, pour R⁴R⁵N-(CH₂)ᵣ (R⁴ et R⁵, qui peuvent être identiques ou différents, représentant des atomes d'hydrogène et/ou des restes alkyle en C₁-C₃ et r étant un nombre de 2 à 4) ou pour -CH(COOH)-CH₂-COOH, et
R³ est mis pour un groupement amino ou mono- ou di-(alkyl en C₁-C₃)amino,
b) un groupement acide phosphonique de formule générale III
R⁶-N(CH₂-PO₃H₂)₂ (III)
dans laquelle
R⁶ est mis pour -CH₂-PO₃H₂, -CH₂-COOH ou -(CH₂)ₛ-N(CH₂-PO₃H₂)₂, s étant un nombre de 2 à 6,
c) un groupement imino-bis(acide méthylènephosphonique),
d) un groupement acide éthylènediaminetétraméthylénephosphonique ou
e) un groupement acide azacycloheptanediphosphonique,
X représente un anion à simple charge négative
n est mis pour le nombre 1 ou 2, mais n'est mis que pour le nombre 1 quand R¹ présente une fonction azotée susceptible de coordination sur le métal central Pt,
m est mis pour le nombre 1,
p est mis pour le nombre 0 ou 2, mais n'est mis que pour le nombre 2 quand Pt est présent au degréd'oxydation IV, et
q est mis pour le nombre 1 ou 2,
ou
n est mis pour le nombre 0, m pour le nombre 2 et q pour le nombre 1,
ou de leurs sels avec des acides et des bases acceptables pharmacologiquement, caractérisé en ce qu'on fait réagir un composé de formule générale
PtX₄
dans laquelle X est tel que défini précédemment, avec des acides phosphoniques de formule générale (II) ou (III) ou avec de l'imino-bis(acide méthylènephosphonique), de l'acide éthylènediaminetétraméthylènephosphonique ou de l'acide azacycloheptanediphosphonique, on réduit éventuellement le produit de la réaction et on le transforme au besoin en les sels d'acides et de bases acceptables pharmacologiquement.
